# EUROPÄISCHE PATENTSCHRIFT

(11) **EP 0 486 774 B1**
(45) Veröffentlichungstag und Bekanntmachung des Hinweises auf die Patenterteilung: **26.10.1994**
(21) Anmeldenummer: 91114439.2
(22) Anmeldetag: 28.08.1991
(51) Int. Cl.: A61K 6/00, A61K 6/083

(54) **Befestigungszement**
Adhesive cement
Ciment adhésif

(30) Priorität: 17.11.1990 DE 4036675; 02.04.1991 DE 4110611
(43) Veröffentlichungstag der Anmeldung: 27.05.1992
(73) Patentinhaber: Heraeus Kulzer GmbH, 63450 Hanau (DE)
(72) Erfinder: Heindl, Detlef, Dr., W-6294 Weinbach-Freienfels (DE); Eppinger, Bernhard, W-6290 Weilburg (DE); Brandenstein, Waltraud, W-6350 Bad Nauheim (DE)
(74) Vertreter: Kühn, Hans-Christian

(56) Entgegenhaltungen:
- EP-A- 0 047 097
- EP-A- 0 064 834
- EP-A- 0 116 121
- EP-A- 0 176 777
- EP-A- 0 237 233
- DE-A- 3 441 564

## Beschreibung

Die Erfindung betrifft einen polymerisierbaren dentalen Befestigungszement auf der Basis von Methacrylsäureester, feinteiligem anorganischem Füllstoff und Photoinitiator-System aus α-Diketon und Amin.

Dentale Befestigungszemente werden benutzt, um Inlays, Onlays, Kronen, Brücken, auch sogenannte Klebe- oder Adhäsivbrücken (Maryland-Brücken), Verblendschalen und dergleichen mit der Zahnsubstanz zu verbinden. Neben infolge von Abbindungsvorgängen härtenden Zementen, wie zum Beispiel den Zinkoxid-Phosphat-Zementen, sind zunehmend auch solche in Gebrauch, die durch Polymerisation aushärten. Die polymerisierbaren Befestigungszemente enthalten als Monomere üblicherweise Ester der Acrylsäure beziehungsweise Methacrylsäure, meist einen feinteiligen anorganischen Füllstoff und daneben die Polymerisation auslösende Katalysatoren.

Aus EP-B1 0 064 834 ist ein Klebstoff zum Ankleben eines Gegenstandes an einen Zahn bekannt, der ein Bindemittelharz, verdünnendes Monomer, einen anorganischen Füllstoff in einer Menge von mindestens 20 Gewichts-Prozent und einen Photoinitiator zur Auslösung der Polymerisation bei Bestrahlung mit sichtbarem Licht enthält. Als Photoinitiator wird ein Gemisch aus einem α-Diketon, ausgewählt aus zum Beispiel Campherchinon, Benzil, Biacetyl, 9,10-Phenanthrenchinon und Naphthochinon, und einem Amin, besonders einem Dialkanol- oder Trialkanolamin, eingesetzt. Als Füllstoffe werden anorganische Gläser, wie zum Beispiel Bariumaluminiumsilicatglas und Lithiumaluminiumsilicatglas, bevorzugt.

Nach DE-C2 34 41 564 lassen sich die Metallflächen von Adhäsivbrücken fest, dicht und spaltfrei mit dem Zahnschmelz verbinden, wenn der dafür benutzte Klebstoff neben Methacrylsäureestern und anorganischem Füllstoff aus silanisiertem Siliciumdioxid mit einer Teilchengröße bis zu 0,04 Mikrometer sowohl einen Katalysator für die chemische Kaltpolymerisation (Autopolymerisation) als auch einen Katalysator für die Photopolymerisation enthält.

Aus EP-A 0 176 777 ist ein durch Photopolymerisation auszuhärtendes Dentalmaterial mit einem Photoinitiator aus eine Benzophenon-Gruppe enthaltendem Polyperoxyester und α-Diketon, das beispielsweise ein Gemisch aus 9,10-Phenanthrenchinon und Campherchinon sein kann, bekannt.

Aus Schweiz Monatsschr Zahnmed. Vol. 99, 4/1989, ist ein niedrigviskoser mikrogefüllter Komposit-Zement bekannt, der durch zweistufige Photopolymerisation gehärtet wird und zunächst gelb gefärbt ist und erst durch die Endhärtung seine definitive Farbe erhält. Dieser Zement enthält zwei Initiator-Systeme mit hohem Campherchinon-Anteil für die Photopolymerisation, deren Absorptionsmaxima bei verschiedenen Wellenlängen des sichtbaren Lichtes liegen. Das Anhärten erfolgt mit Licht einer Wellenlänge, die größer als 470 nm ist, das Endhärten mit Licht der Wellenlänge von rund 470 nm. Durch die zunächst von der Zahnfarbe abweichende Farbe des Zements und seine nach dem Anhärten marzipanähnliche Konsistenz läßt sich der Zement gut bearbeiten und ein Überschuß an Zement, ohne daß die Zahnsubstanz beschädigt wird, rasch und sicher entfernen. Dieser Zement eignet sich nicht für Restaurationen mit lichtunzugänglichen Bezirken.

Es ist nun die Aufgabe der Erfindung, einen polymerisierbaren Befestigungszement der eingangs charakterisierten Art zu finden, der zunächst gefärbt, nach der Bestrahlung mit sichtbarem Licht jedoch zahnfarben ist und gut bearbeitet und sicher ausgehärtet werden kann, ohne daß eine zweistufige Photopolymerisation erforderlich ist.

Der die Lösung der Aufgabe darstellende Befestigungszement ist dadurch gekennzeichnet, daß das α-Diketon ein Gemisch aus 0,01 - 1 Gewichts-% Campherchinon und 0,01 - 1 Gewichts-% 9,10-Phenanthrenchinon ist und zusammen mit 0,1 - 1 Gewichts-% Amin das Photoinitiator-System bildet und der Gehalt an anorganischem Füllstoff 40 - 80 Gewichts-% und seine mittlere Teilchengröße 0,04 - 10 Mikrometer beträgt.

Besonders bewährt hat sich der Befestigungszement, wenn die Menge an Campherchinon 0,02 - 0,05 Gewichts-%, die an 9,10-Phenanthrenchinon 0,02 - 0,05 Gewichts-% und die an Amin 0,3 - 0,5 Gewichts-% beträgt.

Das Amin, das als Reduktionsmittel zusammen mit Campherchinon und 9,10-Phenanthrenchinon das durch sichtbares Licht aktivierbare Photoinitiator-System bildet, kann jedes der für diesen Zweck bekannten Amine sein (siehe zum Beispiel GB-B 1 408 265). Bevorzugt wird jedoch das N,N-Bis-(2-hydroxyäthyl)-p-toluidin.

Als günstig hat es sich erwiesen, als weiteren Bestandteil des Photoinitiator-Systems ein Benzilacetal einzusetzen.

Der erfindungsgemäße Befestigungszement zeichnet sich durch eine in deutlichem Kontrast zur Farbe der Zähne stehende gelbe Färbung, eine mittlere Viskosität und dadurch bedingte sehr gute Verarbeitungseigenschaft aus. Nach dem Einsetzen eines einzuzementierenden Zahnersatzteils in eine Zahnkavität beziehungsweise nach dem Anbringen eines mit dem Zahnschmelz zu verklebenden Zahnersatzteils unter Verwendung des erfindungsgemäßen Befestigungszements hebt sich überschüssiger Zement aufgrund seiner gelben Färbung deutlich sichtbar von der Zahnsubstanz und den Zahnersatzteilen ab. Überschüssiger Zement läßt sich gut entfernen, ohne daß dabei die Gefahr besteht, gleichzeitig damit in den Fugen oder Klebspalten befindlichen Zement herauszuziehen. Durch Bestrahlung mit sichtbarem Licht härtet der Zement unter gleichzeitiger Abnahme der gelben Färbung aus. Die Aushärtung des Zements ist beendet, wenn die gelbe Färbung vollständig verschwunden ist. So erlaubt die gelbe Färbung des Zements die visuelle Kontrolle sowohl der Beseitigung eines Überschusses des Befestigungszements als auch seines Aushärtens.

Es muß als überraschend angesehen werden, daß ein Phenanthrenchinon enthaltendes und dadurch gelb gefärbtes polymerisierbares Material durch Bestrahlung mit sichtbarem Licht vollständig entfärbt werden kann; denn ein solches Verhalten konnte im Hinblick auf DE-A1 30 01 616 nicht erwartet werden. Nach DE-A1 30 01 616 besitzen nämlich aus Phenanthrenchinon und Triäthanolamin enthaltenden polymerisierbaren Zubereitungen durch Bestrahlung mit sichtbarem Licht hergestellte Probekörper auch nach intensiver Belichtung eine kanariengelbe Farbe.

Besonders bewährt hat sich der Befestigungszement, wenn er neben dem Photoinitiator-System auch einen Katalysator für die Autopolymerisation enthält. Mit einem solchen Zement läßt sich, da sein Aushärten sowohl chemisch als auch photochemisch erfolgt, Zahnersatz auch in lichtunzugänglichen Bereichen, in denen keine Photopolymerisation möglich ist, fest mit der Zahnsubstanz verbinden.

Für den Zweck der zusätzlichen Autopolymerisation enthält der Befestigungszement vorzugsweise ein organisches Peroxid, zum Beispiel Diacetylperoxid, Dibenzoylperoxid oder Di-tert.-butylperoxid, das zusammen mit dem Amin ein Redox-System bildet. Redox-Systeme dieser Art stellen bekannte Katalysatoren für die Autopolymerisation von Dentalmaterialien auf Acrylat- beziehungsweise Methacrylat-Basis dar.

Der anorganische Füllstoff kann irgendeiner der für die Verwendung in photopolymerisierbaren Dentalmaterialien an sich bekannten Füllstoffe sein. Vorzugsweise ist er zu 55-75 Gewichts-% in dem Befestigungszement enthalten und besteht er aus mikrofeinem Siliciumdioxid mit einer mittleren Teilchengröße von 0,04 - 0,06 Mikrometer oder aus dem ausgehärteten Zement Röntgenopazität und Abrasionsfestigkeit verleihendem Bariumaluminiumsilicatglas mit einer mittleren Teilchengröße von 0,5 - 1,5 Mikrometer.

Sollte es zur Einstellung der Thixotropie des das Bariumaluminiumsilicatglas enthaltenden Befestigungszements erforderlich sein, so kann dem Zement eine geringe Menge an mikrofeinem Siliciumdioxid zugesetzt werden; im allgemeinen wird dabei der Gehalt an mikrofeinem Siliciumdioxid als Thixotropiemittel nicht mehr als 1 Gewichts-%, bezogen auf den Befestigungszement, betragen.

Die anorganischen Füllstoffe werden vorzugsweise in zum Beispiel durch Behandlung mit 3-Methacryloyloxypropyltrimethoxysilan silanisierter Form eingesetzt.

Der Befestigungszement kann als Ester der Methacrylsäure alle Verbindungen dieser Art, die für die Verwendung in Dentalmaterialien geeignet sind, enthalten. Besonders bewährt haben sich jedoch das Bis-[4-(2-hydroxy-3-methacryloyloxypropoxy)-phenyl]-dimethylmethan, auch als Bowen-Monomer oder Bis-GMA bezeichnet (siehe US 3 066 112), das Triäthylenglykoldimethacrylat, das Diurethandimethacrylat aus 2,2,4-Trimethylhexamethylendiisocyanat und 2-Hydroxyäthylmethacrylat oder Gemische aus diesen Monomeren.

Außer Methacrylsäureester, anorganischem Füllstoff und Polymerisationskatalysator können in dem Befestigungszement noch Farbpigmente, Antioxidationsmittel, UV-Stabilisatoren und andere übliche Zusatzstoffe enthalten sein.

Für den Gebrauch hat es sich bewährt, den Befestigungszement, wenn er Methacrylsäureester, anorganischen Füllstoff, Photoinitiator-System und gegebenenfalls gewünschte Zusatzstoffe enthält, als lagerfähiges Einkomponenten-Material in Pasten-Form zur Verfügung zu stellen.

Der auch durch Autopolymerisation zu härtende Befestigungszement liegt vorzugsweise als pastenförmiges Zweikomponenten-Material vor, wobei zweckmäßigerweise die Zusammensetzung der einen Paste der des Einkomponenten-Materials entspricht und die andere Paste neben Methacrylsäureester und anorganischem Füllstoff den Katalysator für die Autopolymerisation, das Peroxid, enthält.

Der Befestigungszement gemäß der Erfindung eignet sich zur Befestigung jeglicher Art von aus Keramik, Glaskeramik oder Composite-Material gefertigtem Zahnersatz und von Klebebrücken an der Zahnsubstanz.

Soll zum Beispiel eine Zahnkavität mit einem aus einem Composite-Material gefertigten Inlay gefüllt werden, so wird zunächst der Befestigungszement auf die Wände der Kavität und auf das Inlay, soweit es in die Kavität reicht, aufgetragen. Dann wird das Inlay in die Kavität gedrückt. Ein Überschuß an Befestigungszement, der sich durch seine gelbe Farbe deutlich sichtbar von der Zahnsubstanz abhebt und eine weiche Konsistenz besitzt, wird mit einem geeigneten Instrument abgenommen, ohne daß dabei die Zahnsubstanz beschädigt und in der Spalte zwischen Kavität und Inlay vorhandener Befestigungszement entfernt wird. Anschließend wird der Befestigungszement mit einem Lichtgerät, wie es zum Beispiel für das Aushärten von photopolymerisierbarem Zahnfüllungsmaterial bekannt ist, so lange bestrahlt, bis die gelbe Färbung verschwunden und damit der Befestigungszement vollständig ausgehärtet ist.

Zur näheren Erläuterung wird in den folgenden Beispielen die Zusammensetzung von drei Befestigungszementen gemäß der Erfindung beschrieben.

### Beispiel 1

Durch Photopolymerisation auszuhärtender Befestigungszement in Form einer Paste

| | Gewichts-% |
|---|---|
| Bis-GMA | 30,00 |
| Triäthylenglykoldimethacrylat | 16,58 |
| Siliciumdioxid¹, mittlere Teilchengröße 0,04 -0,06 Mikrometer | 51,00 |
| Campherchinon, 10 %ige Lösung in Triäthylenglykoldimethacrylat | 1,00 |
| 9,10-Phenanthrenchinon | 0,02 |
| Benzildimethylacetal², 10 %ige Lösung in Triäthylenglykoldimethacrylat | 0,60 |
| N,N-Bis-(2-hydroxyäthyl)-p-toluidin | 0,40 |
| 2-Hydroxy-4-n-octyloxybenzophenon (UV-Stabilisator) | 0,40 |

| | |
|---|---|
| ¹silanisiert mit 3-Methacryloyloxypropyltrimethoxysilan | |
| ² 1,2-Diphenyl-2,2-dimethoxyäthanon | |

### Beispiel 2

Durch Photopolymerisation auszuhärtender Befestigungszement in Form einer Paste

| | Gewichts-% |
|---|---|
| Bis-GMA | 13,51 |
| Triäthylenglykoldimethacrylat | 7,28 |
| Bariumaluminiumsilicatglas¹, mittlere Teilchengröße 0,7 Mikrometer | 76,79 |
| Campherchinon, 10 %ige Lösung in Triäthylenglykoldimethacrylat | 1,00 |
| 9,10-Phenanthrenchinon Benzildimethylacetal², 10 %ige Lösung in | 0,02 |
| Triäthylenglykoldimethacrylat | 0,60 |
| N,N-Bis-(2-hydroxyäthyl)-p-toluidin | 0,40 |
| 2-Hydroxy-4-n-octyloxybenzophenon (UV-Stabilisator) | 0,40 |

| | |
|---|---|
| ¹silanisiert mit 3-Methacryloyloxypropyltrimethoxysilan | |
| ² 1,2-Diphenyl-2,2-dimethoxyäthanon | |

### Beispiel 3

Durch Photopolymerisation und Autopolymerisation auszuhärtender Befestigungszement in Form zweier Pasten

| Paste A | |
|---|---|
| | Gewichts-% |
| Bis-GMA | 13,51 |
| Triäthylenglykoldimethacrylat Bariumaluminiumsilicatglas¹, mittlere Teilchengröße | 7,28 |
| 0,7 Mikrometer | 76,79 |
| Campherchinon, 10 %ige Lösung in Triäthylenglykoldimethacrylat | 1,00 |
| 9,10-Phenanthrenchinon Benzildimethylacetal², 10 %ige Lösung in | 0,02 |
| Triäthylenglykoldimethacrylat | 0,60 |
| N,N-Bis-(2-hydroxyäthyl)-p-toluidin | 0,40 |
| 2-Hydroxy-4-n-octyloxybenzophenon (UV-Stabilisator) | 0,40 |

| | |
|---|---|
| ¹silanisiert mit 3-Methacryloyloxypropyltrimethoxysilan | |
| ² 1,2-Diphenyl-2,2-dimethoxyäthanon | |

| Paste B | |
|---|---|
| | Gewichts-% |
| Diurethandimethacrylat aus 1 mol 2,2,4-Trimethylhexamethylendiisocyanat und 2 mol 2-Hydroxyäthylmethacrylat | 15,33 |
| Triäthylenglykoldimethacrylat | 6,58 |
| Bariumaluminiumsilicatglas¹, mittlere Teilchengröße 0,7 Mikrometer | 77,12 |
| Siliciumdioxid,¹ mittlere Teilchengröße 0,04-0,06 Mikrometer | 0,53 |
| Dibenzoylperoxid | 0,44 |

| | |
|---|---|
| ¹silanisiert mit 3-Methacryloyloxypropyltrimethoxysilan | |
| ² 1,2-Diphenyl-2,2-dimethoxyäthanon | |

Vor Gebrauch werden die Pasten A und B im Verhältnis 1:1 miteinander vermischt.

## Patentansprüche

1. Polymerisierbarer dentaler Befestigungszement auf der Basis von Methacrylsäureester, feinteiligem anorganischem Füllstoff und Photoinitiator-System aus α-Diketon und Amin, dadurch gekennzeichnet, daß das α-Diketon ein Gemisch aus 0,01 - 1 Gewichts-% Campherchinon und 0,01 - 1 Gewichts-% 9,10-Phenanthrenchinon ist und zusammen mit 0,1 -1 Gewichts-% Amin das Photoinitiator-System bildet und der Gehalt an anorganischem Füllstoff 40 - 80 Gewichts-% und seine mittlere Teilchengröße 0,04 - 10 Mikrometer beträgt.

2. Befestigungszement nach Anspruch 1, dadurch gekennzeichnet, daß er 0,02 - 0,05 Gewichts-% Campherchinon, 0,02 - 0,05 Gewichts-% 9,10-Phenanthrenchinon und 0,3 - 0,5 Gewichts-% Amin enthält.

3. Befestigungszement nach Anspruch 1 oder 2, dadurch gekennzeichnet, daß er zusätzlich ein Benzilacetal in einer Menge von 0,02 - 0,1 Gewichts-% enthält.

4. Befestigungszement nach einem der Ansprüche 1 bis 3, dadurch gekennzeichnet, daß er zusätzlich 0,2 - 0,5 Gewichts-% eines organischen Peroxids enthält.

5. Befestigungszement nach einem der Ansprüche 1 bis 4, dadurch gekennzeichnet, daß der Füllstoff-Gehalt 55 - 75 Gewichts-% beträgt.

6. Befestigungszement nach einem der Ansprüche 1 bis 5, dadurch gekennzeichnet, daß die Füllstoff-Teilchen silanisiert sind.

7. Befestigungszement nach einem der Ansprüche 1 bis 6, dadurch gekennzeichnet, daß der Füllstoff aus Siliciumdioxid mit einer mittleren Teilchengröße von 0,04 - 0,06 Mikrometer besteht.

8. Befestigungszement nach einem der Ansprüche 1 bis 6, dadurch gekennzeichnet, daß der Füllstoff aus Bariumaluminiumsilicatglas mit einer mittleren Teilchengröße von 0,5 - 1,5 Mikrometer besteht.

9. Befestigungszement nach einem der Ansprüche 1 bis 8, dadurch gekennzeichnet, daß er als Methacrylsäureester Bis-[4-(2-hydroxy-3-methacryloyloxypropoxy)-phenyl]-dimethylmethan, Triäthylenglykoldimethacrylat und/oder das Diurethandimethacrylat aus 2,2,4-Trimethylhexamethylendiisocyanat und 2-Hydroxyäthylmethacrylat enthält.

## Claims

1. A polymerizable dental securing cement based on methacrylic acid ester, fine-particulate inorganic filler and a photoinitiator system of α-diketone and amine, characterised in that the α-diketone is a mixture of 0.01 - 1 % by weight camphor quinone and 0.01-1% by weight 9,10-phenanthrene quinone and together with 0.1 - 1 % by weight amine forms the photoinitiator system, and the inorganic filler content amounts to 40 - 80 % by weight and its mean particle size amounts to 0.04 - 10 micrometres.

2. A securing cement according to Claim 1, characterised in that it contains 0.02 - 0.05 % by weight camphor quinone, 0.02 - 0.05 % by weight 9,10-phenanthrene quinone and 0.3 - 0.5 % by weight amine.

3. A securing cement according to Claim 1 or 2, characterised in that it contains in addition a benzil acetal in a quantity of 0.02 - 0.1 % by weight.

4. A securing cement according to one of Claims 1 to 3, characterised in that it contains in addition 0.2 - 0.5 % by weight of an organic peroxide.

5. A securing cement according to one of Claims 1 to 4, characterised in that the filler content amounts to 55 - 75 % by weight.

6. A securing cement according to one of Claims 1 to 5, characterised in that the filler particles are silanised.

7. A securing cement according to one of Claims 1 to 6, characterised in that the filler consists of silicon dioxide with a mean particle size of 0.04 - 0.06 micrometres.

8. A securing cement according to one of Claims 1 to 6, characterised in that the filler consists of barium aluminium silicate glass with a mean particle size of 0.5 - 1.5 micrometres.

9. A securing cement according to one of Claims 1 to 8, characterised in that it contains as methacrylic acid ester bis-[4-(2-hydroxy-3-methacryloyloxypropoxy)-phenyl]-dimethylmethane, triethylene glycol dimethacrylate and/or the diurethanedimethacrylate of 2,2,4-trimethylhexamethylene diisocyanate and 2-hydroxyethylmethacrylate.

## Revendications

1. Ciment de scellement dentaire polymérisable à base d'ester d'acide méthacrylique, de charge minérale fine et d'un système photo-initiateur à base d'α -dicétone et d'amine, caractérisé en ce que l'α-dicétone est un mélange de 0,01-1 % en poids de camphoquinone et de 0,01-1 % en poids de 9,10-phénanthrènequinone et forme le système photo-initiateur avec 0,1-1 % en poids d'amine et la teneur en charge minérale représente 40-80 % en poids et sa granulométrie moyenne est 0,04-10 µm.

2. Ciment de scellement selon la revendication 1, caractérisé en ce qu'il contient 0,02-0,05 % en poids de camphoquinone, 0,02-0,05 % en poids de 9,10-phénanthrènequinone et 0,3-0,5 % en poids d'amine.

3. Ciment de scellement selon la revendication 1 ou 2, caractérisé en ce qu'il contient en plus un acétal de benzile en une quantité de 0,02-0,1 % en poids.

4. Ciment de scellement selon l'une des revendications 1 à 3, caractérisé en ce qu'il contient en plus 0,2-0,5 % en poids d'un peroxyde organique.

5. Ciment de scellement selon l'une des revendications 1 à 4, caractérisé en ce que la teneur en charge est 55-75 % en poids.

6. Ciment de scellement selon l'une des revendications 1 à 5, caractérisé en ce que les particules de charge sont silanisées.

7. Ciment de scellement selon l'une des revendications 1 à 6, caractérisé en ce que la charge de dioxyde de silicium est constituée d'une granulométrie moyenne de 0,04-0,06 µm.

8. Ciment de scellement selon l'une des revendications 1 à 6, caractérisé en ce que la charge de verte de silicate d'aluminium et de baryum a une granulométrie de 0,5-1,5 µm.

9. Ciment de scellement selon l'une des revendications 1 à 8, caractérisé en ce qu'il contient comme ester d'acide méthacrylique du bis-[4(-2-hydroxy-3-méthacrylolyoxypropoxy)-phényl]-diméthylméthane, du diméthacrylate de triéthylèneglycol et/ou le diméthacrylate de diuréthane obtenu à partir de 2,2,4-triméthylhexaméthylènediisocyanate et du méthacrylate de 2-hydroxyéthyle.
